(19) **Europäisches Patentamt / European Patent Office / Office européen des brevets**

(11) **EP 4 504 283 B1**

(12) **EUROPEAN PATENT SPECIFICATION**

(45) Date of publication and mention
of the grant of the patent:
**18.03.2026 Bulletin 2026/12**

(21) Application number: **23715508.0**

(22) Date of filing: **29.03.2023**

(51) International Patent Classification (IPC):
***A61M 1/06*** (2006.01)

(52) Cooperative Patent Classification (CPC):
**A61M 1/06; A61M 1/064; A61M 1/069;
A61M 1/0693;** A61M 2205/051; A61M 2205/368

(86) International application number:
**PCT/EP2023/058077**

(87) International publication number:
**WO 2023/194165 (12.10.2023 Gazette 2023/41)**

(54) **A BREAST PUMP AND A COMPUTER PROGRAM FOR A BREAST PUMP**

BRUSTPUMPE UND EIN COMPUTERPROGRAMM FÜR EINE BRUSTPUMPE

TIRE-LAIT ET UN LOGICIELLE POUR UN TIRE-LAIT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **05.04.2022 EP 22166768**

(43) Date of publication of application:
**12.02.2025 Bulletin 2025/07**

(73) Proprietor: **Koninklijke Philips N.V.**
**5656 AG Eindhoven (NL)**

(72) Inventors:
• **BOURQUIN, Yannyk Parulian Julian**
**5656 AG Eindhoven (NL)**

• **VARGHESE, Babu**
**5656 AG Eindhoven (NL)**
• **BARAGONA, Marco**
**5656 AG Eindhoven (NL)**
• **FRACKOWIAK, Bruno Jean François**
**5656 AG Eindhoven (NL)**
• **PALERO, Jonathan Alambra**
**5656AG Eindhoven (NL)**

(74) Representative: **Philips Intellectual Property &
Standards
High Tech Campus 52
5656 AG Eindhoven (NL)**

(56) References cited:
**US-A1- 2018 147 331    US-A1- 2018 289 874
US-A1- 2020 078 503**

**Description**

FIELD OF THE INVENTION

This invention relates to breast pumps.

BACKGROUND OF THE INVENTION

Breast pumps are used by breast feeding women to extract milk from their breast such that the extracted milk can be fed to their babies at a later time.

It is well known that the best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup, called a breast shield, and a vacuum is applied such that milk is extracted. A motorized breast pump typically has two operating modes, namely a stimulation mode and an extraction mode. The operation of a typical breast pump makes use of a cyclic pressure waveform, typically a negative pressure or vacuum, which is applied to the breast and nipple within a breast shield (or a pair of breast shields) to draw the milk from the nipple into a collection container. The typical pressure profiles oscillate between a maximum negative pressure and then return to atmospheric pressure at the end of each cycle.

During the stimulation mode, the milk ejection reflex is stimulated. This for example makes use of a relatively high frequency cyclic pressure waveform such as 2Hz. Once milk comes out of the breast it is advised to switch to the extraction mode, which has a lower frequency (typically around 1Hz) and higher intensity pumping (i.e. lower under pressure) for more effective milk extraction.

When the breast pump is activated, a pressure source such as a vacuum pump inside the breast pump generates a vacuum and the stimulation mode can be started. The vacuum pump is typically driven by an electric motor. For portable breast pumps, these motors are driven by battery power. There are also breast pumps using manually driven mechanical pumps.

US 2018/0147331A1 discloses an electric breast pump. The electric breast pump includes a conduit module, a suction module and a host. The conduit module includes a connector, a first conduit and a second conduit. The connector has an electric contact and an opening hole, and the connector is further disposed with a protrusion at the side of the connector on which the opening hole is formed. The first end of the first conduit connects to the electric contact. The first end of the second conduit connects to the opening hole. The suction module has an adapter body coupled with the connector to connect the suction module and the conduit module. The host connects to the second end of the first conduit and the second end of the second conduit. The host controls the suction module via the conduit module to extract and collect milk when the suction module contacts mother's breast.

US 2018/0289874A1 is about a photo therapy apparatus comprising one or more light sources which are integrated with a breast pump for providing photo therapy treatment to the breast and nipple of women during pumping of breast milk. The light sources can be lasers, light emitting diodes (LEDs), lamps, or any other types of light sources which produce therapeutic light in a desired wavelength range. The therapeutic light is delivered through the breast-shield or flange of the breast pump to the breast and nipple and produces a variety of photochemical processes, e.g. increase of ATP (adenosine triphosphate ), triggering of photo neurological response and activation of enzymes, changes in local pressure, increases in temperature, and deformation of cellular membranes. Such photochemical reaction helps to reduce pain and promote healing of nipple lesion and in the meantime enhance yield of breast milk.

US 2020/0078503A1 discloses automatically adjusting various pumping parameters based on data obtained from one or more sensors. The one or more sensors can produce data regarding an amount of milk expressed by a user. The adjusting of the pumping parameters can be configured to, for example, help the user efficiently express milk by utilizing a closed-feedback system that monitors the flow rate of the expressed milk.

Recently, there is a trend towards wearable breast pumps. These devices generally have a smaller form factor and can be fully worn on the user's body, allowing the user to move around freely. Some of these are small enough to fit into a user's bra. A motor, battery and milk collection container are for example integrated to form a single unit.

For mothers, it is important that using a breast-pump is efficient and comfortable. Unfortunately, for a lot of mothers, the breast pump is not efficient and may feel uncomfortable. It is known that adding warmth to the breast can provide comfort and increase milk expression efficiency. For example, it has been reported that a warm breast shield at 39 degrees Celsius improves milk expression.

However, implementing tissue heating in a breast pump is challenging due to the movement of the breast (as a result of the pressure profile of the breast pump) and this can result in ineffective heating or hot spots.

SUMMARY OF THE INVENTION

The invention is defined by the claims.

According to examples in accordance with an aspect of the invention, there is provided a breast pump comprising:

a breast shield;
a pressure source for applying a pressure profile to a breast received in the breast shield;
an RF signal source for generating RF signals;
an electrode arrangement for direct or indirect contact with the breast and to which the RF signals are supplied, for providing RF heating of the breast; and
a controller for controlling the supply of the RF signals to the electrode arrangement with timing which depends on the pressure profile.

This breast pump incorporates an RF heating system to provide warmth to the breast during milk expression. The tissue heating has been found to make the use of the breast pump more comfortable and more efficient. Tissue heating using RF technologies is known for warming up human body tissue, for example it is mainly use in the field of skin rejuvenation. Compared to general contact methods for generating warmth, the warmth generated by RF heating penetrates beneath the surface of the skin, and this avoids the need for extensive preparation such as with a warm compress. RF heating also does not require any preparation from the user and it can simply automatically switch on when using the breast pump and allows deeper penetration of the energy.

However, a problem when implementing RF heating is the need to keep good contact between the skin and the RF heating electrodes. This is a particular issue with a breast pump because of the changing conditions in the breast shield due to the cyclic pressure waveform. The cyclic nature of the vacuum profile means the skin contact may vary during pumping. Due to the partial contact of the skin with the RF electrodes, this may induce hot spots on the skin and eventually lead to discomfort.

By operating the heating such that the timing is coordinated with the pressure profile applied by the breast pump, hot spots are avoided for example by ensuring the heating only takes place when there is a negative (vacuum) pressure applied, which results in good electrode contact. Thus, the generation of hot spots is avoided when applying RF heating during breast pumping.

The pressure profile comprises at least two different pressure levels. The pressure profile may toggle between those at least two pressure profiles, or there may be more continuous adjustment between different pressure levels.

The contact may be direct or indirect. In other words, the RF electrode may contact the skin, or they may be an intermediate layer between them, but with no air gaps intended between the breast tissue and the RF electrode so that the heating characteristics are known when that direct or indirect contact is established.

The timing is such that the vacuum pressure applied by the pressure source ensures contact between the breast tissue and the electrode arrangement. The RF heating is for example most effective when the pressure is lowest (i.e. the greatest level of under pressure i.e. vacuum) is applied.

The coordination between the timing of the RF heating and the pressure profile may be direct, in that the pressure is measured or the control signal for the pressure source is used to control the heating. However, it may be indirect, in that another signal is sensed which varies with the use of the breast pump (e.g. sound) and this correlates with the pressure profile.

The RF signals for example lie the range 0.1 MHz to 50MHz. This range is particularly suitable for RF tissue heating. The RF signals for example are for example in the range 1MHz to 10MHz such as around 4MHz.

The electrode arrangement may be attached to or integrated with the breast shield. Thus, the heating electrodes may be integrated as part of the breast pump during manufacture or they may be applied as an upgrade to an existing breast pump.

In a first example, the controller may be adapted to control the supply of the RF signals in dependence on control signals applied to the pressure source.

Thus, a controller for the pressure source (i.e. the main controller of the breast pump) may provide the control signals for use in controlling the delivery of RF heating. There may be a single controller for the pressure source and for the RF heating function, or there may be separate controllers.

The controller may be adapted to receive information relating to the control signals applied to the pressure source:

as a wired connection; or
as a wireless communication.

Thus, the controller for the heating may be integrated (wired) into the breast pump or it may be part of a remote system which receives information from the main controller wirelessly (e.g. over Bluetooth or WiFi).

The breast pump may comprise a pressure sensor for sensing a pressure in the breast shield, wherein the controller is adapted to control the supply of the RF signals in dependence on the pressure sensed by the pressure sensor.

This example is based on pressure sensing, rather than (or as well as) the use of control signals of the pressure source. This is suitable for an integrated heating system or a system which may be added as an upgrade.

The controller is for example adapted to apply one or more pressure threshold values and to control the supply of the RF signals accordingly. Thus, the heating may be activated only when there is a high vacuum level, which indicates that there will be good contact between the electrode arrangement and the skin.

The controller may be adapted to supply RF signals from a first time point when an applied vacuum pressure reaches a first threshold to a later second time point, for example when the vacuum pressure is released. This is one way to time the heating. The heating may only need to be conducted during a fraction of the breast pump pressure profile cycles, but when heating is being applied, it is applied during the periods of those cycles with high vacuum level.

The pressure profile may comprise a first profile for a stimulation mode and a second profile for an expression mode, and the controller is adapted to control the supply of the RF signals in dependence on the mode. For example, there may be a pre-heating mode during the stimulation mode, and a lower power (i.e. lower average time duration) heat maintenance mode during expression. The controller is for example adapted to deliver the RF signals as pulses, wherein a pulse rate is higher during the stimulation mode than the expression mode.

The breast pump may comprise a contact quality sensor for detecting a contact quality between the electrode arrangement and the breast, wherein the controller is adapted to control the supply of the RF signals in dependence on the detected contact quality. The detected contact quality will vary with the pressure profile so that the RF signals are again supplied in dependence on the pressure profile. However, the contact quality itself is measured. The contact quality sensor may be an impedance sensor and it may use the electrode arrangement of the RF heating system. Thus, skin impedance may be measured using the RF electrodes, and the measured impedance is a measure of skin contact and pressure. Thus, skin impedance sensing may be used as a contact quality measurement.

In another example, the breast pump comprises an acoustic sensor, wherein the controller is adapted to determine the operation of the pressure source from the acoustic sensor signals and control the supply of the RF signals in dependence on the determined operation of the pressure source. This is suitable for a separate remote system for the RF heating, for example to be used as an upgrade. It does not need any electrical connection or any signal communication between the breast pump to the heating system, so does not need any adaptation to an existing breast pump.

The invention also provides a computer program comprising computer program code means which is adapted, when said program is run by the controller of the breast pump defined above, to implement a method of controlling the breast pump, comprising:

applying a pressure profile to a breast received in a breast shield using a pressure source; and
supplying RF signals to an electrode arrangement which is for contact with the breast, for providing RF heating of the breast,
wherein the supply of the RF signals to the electrode arrangement is controlled in dependence on the pressure profile.

The RF signals for example lie the range 0.1 MHz to 50MHz.

The supply of the RF signals may be controlled in dependence on:

control signals applied to the pressure source; or
a pressure sensed by a pressure sensor; or
whether the pressure profile is a first profile of a stimulation mode or a second profile of an expression mode; or
a contact quality detected between the electrode arrangement and the breast; or
acoustic sensor signals which sense the operation of the pressure source.

Two or more of these sensing approaches may be combined within a single overall system.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a known breast pump system;
Figure 2 shows a wearable breast pump;
Figure 3 shows a first example of the breast pump with RF heating;
Figure 4 shows a second example of the breast pump with RF heating as a modification to Figure 3;
Figure 5 shows a third example of the breast pump with RF heating as another modification to Figure 3;
Figure 6 shows a fourth example of the breast pump with RF heating as another modification to Figure 3;
Figure 7 shows a fifth example of the breast pump with RF heating as another modification to Figure 3; and
Figure 8 shows one cycle of a breast pump pressure profile as pressure vs. time and shows when heating may be applied.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump having a breast shield, a pressure source for applying a pressure profile to a breast received in the breast shield and an RF signal source for generating RF signals. The RF signals are applied to an electrode arrangement to provide RF heating of the breast. The supply of the RF signals to the electrode arrangement, i.e. the heating, is controlled with timing which depends on the pressure profile. In this way, the heating is provided at times within the pressure profile when the heating function will be most effective, for example at times of maximum under pressure and hence suction of the breast tissue against the breast shield.

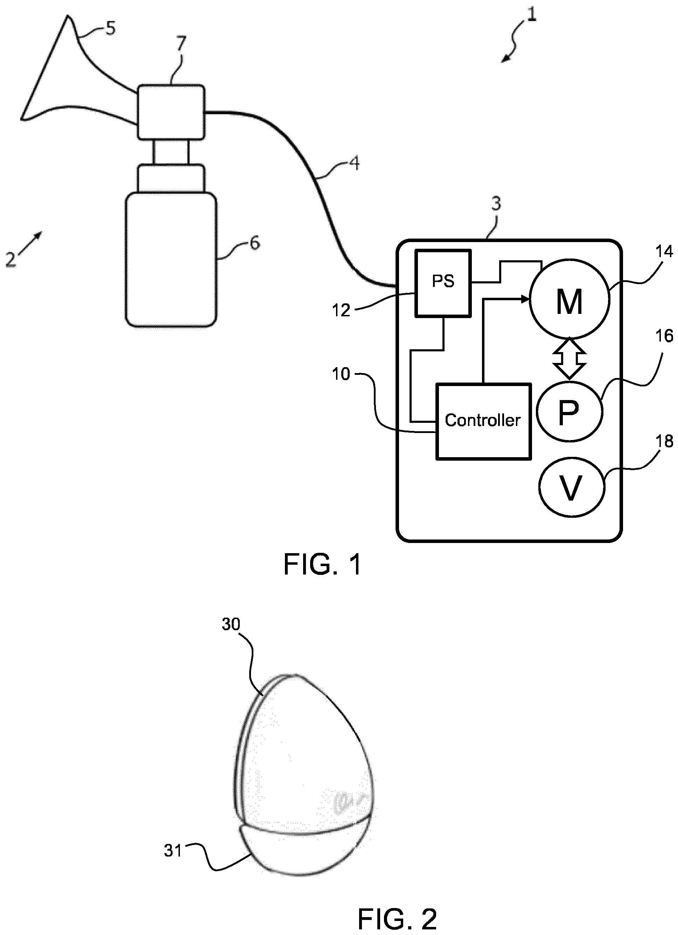

Figure 1 shows a known breast pump system 1, comprising an expression unit 2 and a drive arrangement for controlling the expression function of the breast pump.

In the example shown, the drive arrangement comprises an electric pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a milk collection container 6 for collecting the expressed milk. The funnel 5 and the container 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the diaphragm prevents expressed milk from flowing directly into the pump arrangement 3.

The pump arrangement 3 comprises a controller 10, a power source 12, a motor 14 and a vacuum pump 16 (i.e. the impeller driven by the motor 14). A membrane pump (displacement pump) may instead be used. The controller controls 10 the operation of the power source 12, motor 14 and vacuum pump 16. The pump arrangement 3 further comprises a solenoid valve 18.

In use, the vacuum pump 16 applies a vacuum to the diaphragm located in the main body 7 so that it deforms. The diaphragm deforms to create a vacuum in the funnel 5, when it is sealed to the breast, which in turn applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection container. This enables breast pumping to be performed more discretely and leaves the hands free to do other things.

There are also other types of wearable breast pump whereby the expression unit is fixed to a breast and the pump unit and/or milk collection container are situated elsewhere on the body of a user.

Figure 2 shows a wearable breast pump, comprising an expression kit 30 attached to a respective milk collection container 31.

The invention can be applied to any existing type of breast pump.

The invention makes use of RF tissue heating, by adding an electrode arrangement which contacts the breast. RF signals are supplied to the electrode arrangement for providing RF heating of the breast. The RF heating is controlled with timing which depends on the pressure profile. The tissue heating makes the use of a breast pump more comfortable and more efficient. By operating the heating such that the timing is coordinated with the pressure profile applied by the breast pump, heating takes place when there is good electrode contact, so that hot spots are avoided.

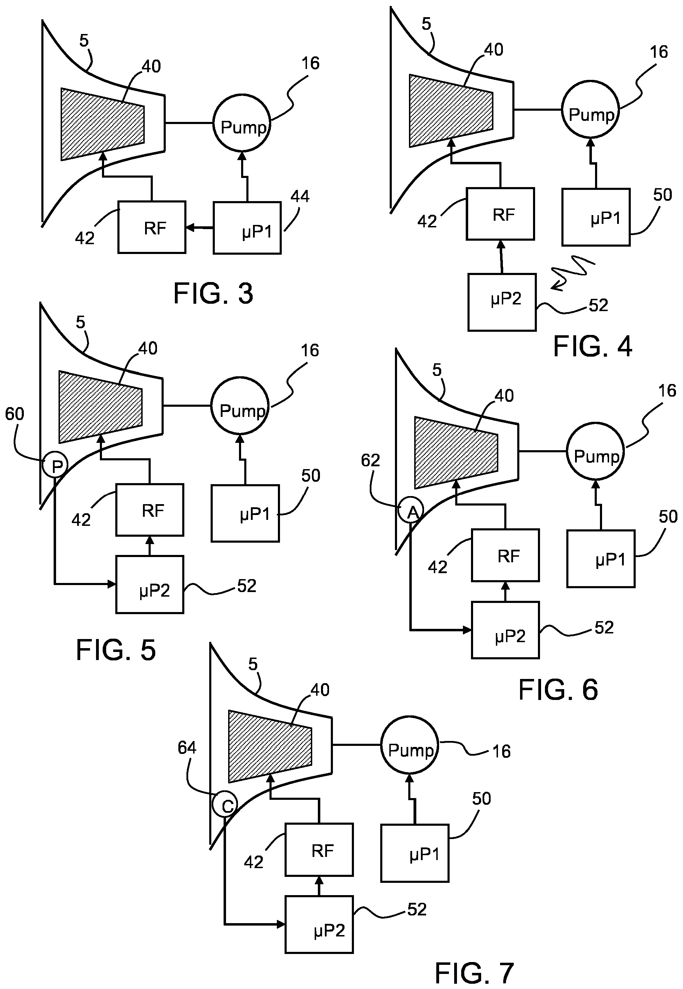

Figure 3 shows a first example of the breast pump with RF heating. For simplicity, only the breast shield 5 and pressure source, i.e. pump 16, of the typical breast pump configuration are shown. An electrode arrangement 40 is provided at the internal surface of the breast shield 5 so that when a vacuum is applied to the space between the breast shield and the breast, the breast is brought into contact with the electrode arrangement 40. A signal source 42 delivers RF signals to the electrode arrangement. The electrode arrangement 40 may be integrated with the breast shield during manufacture or it may be designed as a retrofit feature.

The RF signals for example lie the range 0.1 MHz to 50MHz. This range is particularly suitable for RF tissue heating. The RF signals for example are in the range 1MHz to 10MHz, such as around 4MHz.

The example of Figure 3 has a single controller 44, which functions both as the main controller for the pump 16 and for the RF signal source 42.

The single controller 44 in this example controls the supply of the RF signals in dependence on the control signals which is applies to the pump 16.

Figure 4 shows a second example of the breast pump with RF heating as a modification to Figure 3. In this case, there is a first controller 50 for the pump 16 (i.e. the main breast pump controller) and a second controller 52 for the RF signal source.

The communication between the two controllers 50, 52 may be wired or wireless. The wireless option is shown in Figure 4. It enables the heating system to be a retrofit system, for example only needing a software update of the breast pump to allow the pump control signals to be provided over a wireless communications link such as Bluetooth or WiFi.

Figure 5 shows a third example of the breast pump with RF heating as another modification to Figure 3. In this case, there is again a first controller 50 for the pump 16 and a second controller 52 for the RF signal source. A pressure sensor 60 provides a pressure signal to the second controller 52 so that no modification is needed to the main breast pump functionality.

Figure 6 shows a fourth example of the breast pump with RF heating as another modification to Figure 3. In this case, there is again a first controller 50 for the pump 16 and a second controller 52 for the RF signal source. An acoustic sensor 62 provides an acoustic signal to the second controller 52 so that no modification is needed to the main breast pump functionality. The acoustic signal records the sound of the breast pump and enables the operation of the pump to be identified so that the supply of the RF signals can be controlled accordingly.

Figure 7 shows a fifth example of the breast pump with RF heating as another modification to Figure 3. In this case, there is again a first controller 50 for the pump 16 and a second controller 52 for the RF signal source. A contact sensor 64 provides a contact sensing signal to the second controller 52 again so that no modification is needed to the main breast pump functionality. The contact sensor is for determining a contact quality between the electrode arrangement and the breast. The detected contact quality will vary with the pressure profile so that the RF signals are again supplied in dependence on the pressure profile.

The contact sensing for example comprises an impedance measurement. This impedance measurement may be made using the RF heating electrode arrangement itself. The RF heating may be activated when the sensed impedance is below a pre-set threshold value, indicating good contact. The activation may include an appropriate control scheme (e.g. a delay) to account for short periods of unstable contact.

It can be seen from these examples that the coordination between the timing of the RF heating and the pressure profile may be direct, in that the pressure is measured or the control signal for the pressure source is used to control the heating. However, it may be indirect, in that another signal is sensed which varies with the use of the breast pump (e.g. sound or contact quality) and this signal is correlated with the pressure profile with the same result that the RF heating is timed in a manner which depends on the pressure profile characteristics over time.

When a pressure signal (either measured or as a control signal) is available, pressure threshold values may be applied.

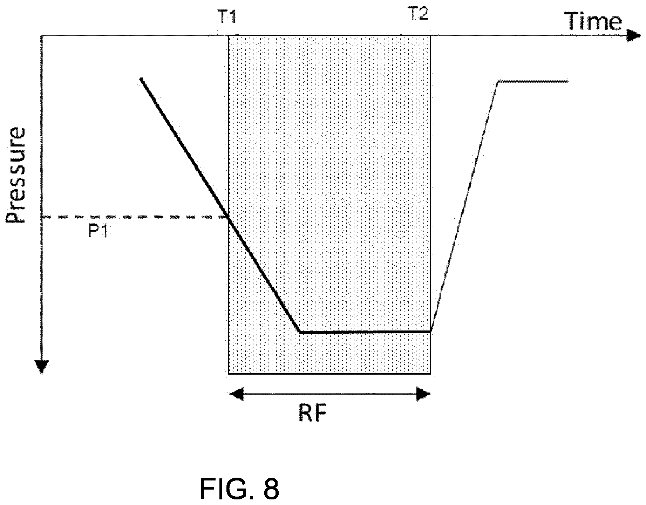

Figure 8 shows one cycle of a breast pump pressure profile as pressure vs. time. The pressure is relative to ambient pressure and hence is negative, indicating that a partial vacuum is applied. RF signals are supplied from a first time point T1 within the pressure profile when the applied vacuum pressure reaches a first threshold P1. The pressure P1 is for example -20mbar (-2kPa). The RF signals end at a second time point T2 when the vacuum pressure is released.

The RF heating may be applied during all or only some of the pressure profile cycles. The vacuum profile may be adjusted to increase or decrease the heating time according to user preferences, for example by changing a part of the pressure profile during which heating is applied, such as the so-called dwell-in time (during which maximum negative pressure is applied).

As explained above, a breast pump may be configured to provide different modes of operation, such as a first pressure profile for a stimulation mode and a second pressure profile for an expression mode. The controller of the heating function may then implement a pre-heating mode during the stimulation mode, and a lower power (i.e. lower average time duration) heat maintenance mode during expression. For this purpose, for a pulsed delivery of RF signals, the pulse rate can be higher during the stimulation mode than the expression mode. For example, the RF signal may be delivered at 100-125 cycles per minute during the stimulation phase such that an initial baseline temperature is reached, and is followed by 40-70 cycles per minute during expression phase, which requires only low settings in order to maintain the temperature.

The measures above may be combined to improve the safety of the RF treatment. In particular, using both the pump setting and the RF contact sensing would allow to determine at which pressure level the contact is best for a specific user in order to personalize the treatment.

Various electrode designs may be used. One approach is to use concentric circular electrodes (e.g. two such electrodes) around the breast with a same area. A maximal temperature may be set at 42 degrees Celsius to prevent discomfort and pain. A temperature sensor may be incorporated in any of the designs above for temperature monitoring. A power delivered to the tissue is for example of the order of ones of Watts, e.g. 1.25W Assuming an RF efficiency of approximately 70%, a total power of around 1.8 W is for example suitable. Thus, the RF heating may be battery operated.

Alternatively, two electrodes may be positioned on the two sides of the breast shield formed as stripes, for example with widths in the range 1cm to 3cm. Compared to circular electrodes, a deeper treatment in the breast can be achieved with less thermal gradient towards the nipple. Moreover, the distance between the electrodes influences the heating. If the distance is large, the current is spread over a larger volume of breast tissue, leading to less heating per unit volume and a lower heat penetration in the breast tissue. On the other hand, for a lower distance between electrodes, the current becomes more concentrated, leading to a stronger heat penetration in the breast tissue. Therefore, the distance between electrodes can be adjusted as a design parameter to provide sufficient heat while preventing discomfort and pain to the user.

Hot spots on the breast surface can be reduced with particular electrode geometries, for example a toroidal geometry. Segmented electrodes may also be used.

The RF frequency is selected to provide optimal breast heating. It has for example been found that there is a significant increase of the heating of breast tissue when increasing the frequency from around 1MHz up to 4 MHz.

When there are cyclic expression and stimulation modes, they for example have different cyclic frequency and different maximum under-pressure (i.e. vacuum) levels. The expression mode for example cycles between a baseline vacuum and a maximum vacuum (i.e. a maximum under-pressure) "Max_Vac".

There is first a time to (maximum) vacuum, TTV, then a dwell-in time DI, then a time to atmosphere (or to the baseline vacuum), TTA, and finally a dwell-out time, DO. The vacuum rate is defined as Max_Vac/TTV and the atmospheric rate is defined as Max_Vac/TTA.

By way of example:

Baseline _Vac = atmospheric pressure
Max_Vac = 300 mbar (30 kPa) below atmospheric pressure

$$TTV = 0.85 \text{ s}$$

$$DI = 0.3 \text{ s}$$

$$TTA = 0.05 \text{ s}$$

$$DO = 0.35 \text{ s}$$

This gives a total cycle time T _cycle of 1.55s.

These values are simply by way of example. The maximum under-pressure may be higher, for example 350 mbar (35 kPa) and the durations may differ. The cycle time is generally of the order of 1 to 2 seconds, typically 1 second (1 Hz).

The stimulation mode for example has a faster frequency hence with a shorter cycle time of around 0.5 seconds (2 Hz), and a lower vacuum, for example a maximum under-pressure level of 150 mbar (15 kPa).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump comprising:

   a breast shield (5);
   a pressure source (16) for applying a pressure profile to a breast received in the breast shield,
   an RF signal source (42) for generating RF signals;
   and **characterized by** an electrode arrangement (40) for direct or indirect contact with the breast and to which the RF signals are supplied, for providing RF heating of the breast; and
   a controller (44, 52) for controlling the supply of the RF signals to the electrode arrangement with timing which depends on the pressure profile.

2. The breast pump of claim 1, wherein the RF signals lie the range 0.1 MHz to 50 MHz.

3. The breast pump of claim 1 or 2, wherein the electrode arrangement (40) is attached to or integrated with the breast shield.

4. The breast pump of any one of claims 1 to 3, wherein the controller (44, 52) is adapted to control the supply of the RF signals in dependence on control signals applied to the pressure source.

5. The breast pump of claim 4, wherein the controller is adapted to receive information relating to the control signals applied to the pressure source:

   as a wired connection; or
   as a wireless communication.

**6.** The breast pump of any one of claims 1 to 5, comprising a pressure sensor (60) for sensing a pressure in the breast shield, wherein the controller is adapted to control the supply of the RF signals in dependence on the pressure sensed by the pressure sensor.

**7.** The breast pump of any one of claims 4 to 6, wherein the controller is adapted to apply one or more pressure threshold values and to control the supply of the RF signals accordingly.

**8.** The breast pump of claim 7, wherein the controller is adapted to supply RF signals from a first time point (T1) when an applied vacuum pressure reaches a first threshold to a later second time point (T2).

**9.** The breast pump of any one of claims 1 to 8, wherein the pressure profile comprises a first profile for a stimulation mode and a second profile for an expression mode, and the controller is adapted to control the supply of the RF signals in dependence on the mode.

**10.** The breast pump of claim 9, wherein the controller is adapted to deliver the RF signals as pulses, wherein a pulse rate is higher during the stimulation mode than the expression mode.

**11.** The breast pump of any one of claims 1 to 10, comprising a contact quality sensor (64) for detecting a contact quality between the electrode arrangement and the breast, wherein the controller is adapted to control the supply of the RF signals in dependence on the detected contact quality.

**12.** The breast pump of any one of claims 1 to 3, comprising an acoustic sensor (62), wherein the controller is adapted to determine the operation of the pressure source from the acoustic sensor signals and control the supply of the RF signals in dependence on the determined operation of the pressure source.

**13.** A computer program comprising computer program code means which is adapted, when said program is run by the controller of the breast pump of any one of claims 1 to 12, to implement a method of controlling the breast pump, comprising:

applying a pressure profile to a breast received in a breast shield using a pressure source; and
supplying RF signals to an electrode arrangement which is for contact with the breast, for providing RF heating of the breast,
wherein the supply of the RF signals to the electrode arrangement is controlled in dependence on the pressure profile.

**14.** The computer program of claim 13, wherein RF signals lie the range 0.1 MHz to 50MHz.

**15.** The computer program of claim 13 or 14, wherein the method comprises controlling the supply of the RF signals in dependence on:

control signals applied to the pressure source; or
a pressure sensed by a pressure sensor; or
whether the pressure profile is a first profile of a stimulation mode or a second profile of an expression mode; or
a contact quality detected between the electrode arrangement and the breast; or
acoustic sensor signals which sense the operation of the pressure source.

**Patentansprüche**

**1.** Milchpumpe, umfassend:

einen Brustschild (5);
eine Druckquelle (16) zum Anlegen eines Druckprofils an eine im Brustschild befindliche Brust,
eine HF-Signalquelle (42) zum Erzeugen von HF-Signalen;
und **gekennzeichnet durch**
eine Elektrodenanordnung (40) zum direkten oder indirekten Kontakt mit der Brust, und der die HF-Signale zum Bereitstellen einer HF-Erwärmung der Brust zugeführt werden; und
eine Steuereinheit (44, 52) zum Steuern der Zufuhr der HF-Signale zur Elektrodenanordnung mit einer Zeitsteuerung, die vom Druckprofil abhängt.

**2.** Milchpumpe nach Anspruch 1, wobei die HF-Signale im Bereich von 0,1 MHz bis 50 MHz liegen.

**3.** Milchpumpe nach Anspruch 1 oder 2, wobei die Elektrodenanordnung (40) am Brustschild angebracht oder darin integriert ist.

**4.** Milchpumpe nach einem der Ansprüche 1 bis 3, wobei die Steuereinheit (44, 52) angepasst ist, um die Zufuhr der HF-Signale in Abhängigkeit von den an die Druckquelle angelegten Steuersignalen zu steuern.

**5.** Milchpumpe nach Anspruch 4, wobei die Steuereinheit angepasst ist, um Informationen in Bezug auf die an die Druckquelle angelegten Steuersignale zu empfangen:

als kabelgebundene Verbindung; oder
als drahtlose Kommunikation.

**6.** Milchpumpe nach einem der Ansprüche 1 bis 5, umfassend einen Drucksensor (60) zum Erfassen eines Drucks im Brustschild, wobei die Steuereinheit angepasst ist, um die Zufuhr der HF-Signale in Abhängigkeit von dem vom Drucksensor erfassten Druck zu steuern.

**7.** Milchpumpe nach einem der Ansprüche 4 bis 6, wobei die Steuereinheit angepasst ist, um einen oder mehrere Druckschwellenwerte anzuwenden und um die Zufuhr der HF-Signale dementsprechend zu steuern.

**8.** Milchpumpe nach Anspruch 7, wobei die Steuereinheit angepasst ist, um HF-Signale von einem ersten Zeitpunkt (T1), wenn ein angelegter Vakuumdruck einen ersten Schwellenwert erreicht, bis zu einem späteren zweiten Zeitpunkt (T2) zuzuführen.

**9.** Milchpumpe nach einem der Ansprüche 1 bis 8, wobei das Druckprofil ein erstes Profil für einen Stimulationsmodus und ein zweites Profil für einen Abpumpmodus umfasst und die Steuereinheit angepasst ist, um die Zufuhr der HF-Signale in Abhängigkeit vom Modus zu steuern.

**10.** Milchpumpe nach Anspruch 9, wobei die Steuereinheit angepasst ist, um die HF-Signale als Impulse abzugeben, wobei eine Impulsrate im Stimulationsmodus höher ist als im Abpumpmodus.

**11.** Milchpumpe nach einem der Ansprüche 1 bis 10, umfassend einen Kontaktqualitätssensor (64) zur Erfassung einer Kontaktqualität zwischen der Elektrodenanordnung und der Brust, wobei die Steuereinheit angepasst ist, um die Zufuhr der HF-Signale in Abhängigkeit von der erfassten Kontaktqualität zu steuern.

**12.** Milchpumpe nach einem der Ansprüche 1 bis 3, umfassend einen akustischen Sensor (62), wobei die Steuereinheit angepasst ist, um den Betrieb der Druckquelle aus den Signalen des akustischen Sensors zu bestimmen und die Zufuhr der HF-Signale in Abhängigkeit vom bestimmten Betrieb der Druckquelle zu steuern.

**13.** Computerprogramm, das Computerprogrammcodemittel umfasst, die, wenn das Programm von der Steuereinheit der Milchpumpe nach einem der Ansprüche 1 bis 12 ausgeführt wird, angepasst sind, um ein Verfahren zum Steuern der Milchpumpe zu implementieren, umfassend:

Anlegen eines Druckprofils an eine im Brustschild befindliche Brust, unter Verwendung einer Druckquelle; und
Zuführen von HF-Signalen zu einer Elektrodenanordnung die zur Kontaktaufnahme mit der Brust, zum Bereitstellen von HF-Wärme der Brust dient,
wobei die Zufuhr der HF-Signale zur Elektrodenanordnung in Abhängigkeit vom Druckprofil gesteuert wird.

**14.** Computerprogramm nach Anspruch 13, wobei HF-Signale im Bereich von 0,1 MHz bis 50MHz liegen.

**15.** Computerprogramm nach Anspruch 13 oder 14, wobei das Verfahren Steuern der Zufuhr der HF-Signale umfasst, in Abhängigkeit von:

Steuersignalen, die an die Druckquelle angelegt werden; oder
einem von einem Drucksensor erfassten Druck; oder
ob das Druckprofil ein erstes Profil eines Stimulationsmodus oder ein zweites Profil eines Abpumpmodus ist; oder
einer zwischen der Elektrodenanordnung und der Brust erfassten Kontaktqualität; oder
akustischen Sensorsignalen, die den Betrieb der Druckquelle erfassen.

**Revendications**

**1.** Tire-lait comprenant :

une téterelle (5) ;
une source de pression (16) pour appliquer un profil de pression à un sein reçu dans la téterelle,
une source de signal RF (42) pour générer des signaux RF ;
et **caractérisé par**
un agencement d'électrodes (40) destiné au contact direct ou indirect avec le sein et auquel sont fournis les signaux RF, pour assurer le chauffage RF du sein ; et
un dispositif de commande (44, 52) pour commander l'alimentation des signaux RF à l'agencement d'électrodes avec une synchronisation qui dépend du profil de pression.

**2.** Tire-lait selon la revendication 1, dans lequel les signaux RF se situent dans la plage de 0,1 MHz à 50 MHz.

**3.** Tire-lait selon la revendication 1 ou 2, dans lequel l'agencement d'électrodes (40) est fixé ou intégré à la téterelle.

**4.** Tire-lait selon l'une quelconque des revendications 1 à 3, dans lequel le dispositif de commande (44, 52) est adapté pour commander l'alimentation des signaux RF en fonction de signaux de commande appliqués à la source de pression.

5. Tire-lait selon la revendication 4, dans lequel le dispositif de commande est adapté pour recevoir des informations relatives aux signaux de commande appliqués à la source de pression :

sous la forme d'une connexion câblée ; ou
sous la forme d'une communication sans fil.

6. Tire-lait selon l'une quelconque des revendications 1 à 5, comprenant un capteur de pression (60) pour détecter une pression dans la téterelle, dans lequel le dispositif de commande est adapté pour commander l'alimentation des signaux RF en fonction de la pression détectée par le capteur de pression.

7. Tire-lait selon l'une quelconque des revendications 4 à 6, dans lequel le dispositif de commande est adapté pour appliquer une ou plusieurs valeurs de seuil de pression et pour commander la fourniture des signaux RF en conséquence.

8. Tire-lait selon la revendication 7, dans lequel le dispositif de commande est adapté pour fournir des signaux RF à partir d'un premier point temporel (T1) auquel une pression de vide appliquée atteint un premier seuil jusqu'à un second point temporel ultérieur (T2).

9. Tire-lait selon l'une quelconque des revendications 1 à 8, dans lequel le profil de pression comprend un premier profil pour un mode de stimulation et un second profil pour un mode d'expression, et le dispositif de commande est adapté pour commander la fourniture des signaux RF en fonction du mode.

10. Tire-lait selon la revendication 9, dans lequel le dispositif de commande est adapté pour délivrer les signaux RF sous forme d'impulsions, dans lequel une fréquence d'impulsions est plus élevée pendant le mode de stimulation que pendant le mode d'expression.

11. Tire-lait selon l'une quelconque des revendications 1 à 10, comprenant un capteur de qualité de contact (64) pour détecter une qualité de contact entre l'agencement d'électrodes et le sein, dans lequel le dispositif de commande est adapté pour commander la fourniture des signaux RF en fonction de la qualité de contact détectée.

12. Tire-lait selon l'une quelconque des revendications 1 à 3, comprenant un capteur acoustique (62), dans lequel le dispositif de commande est adapté pour déterminer le fonctionnement de la source de pression à partir des signaux de capteur acoustique et commander la fourniture des signaux RF en fonction du fonctionnement déterminé de la source de pression.

13. Programme informatique comprenant des moyens de code de programme informatique qui sont adaptés, lorsque le programme est exécuté par le dispositif de commande du tire-lait selon l'une quelconque des revendications 1 à 12, pour mettre en œuvre un procédé de commande du tire-lait, comprenant :

l'application d'un profil de pression à un sein reçu dans une téterelle à l'aide d'une source de pression ; et
la fourniture de signaux RF à un agencement d'électrodes destiné à être en contact avec le sein, afin d'assurer un chauffage RF du sein, dans lequel la fourniture des signaux RF à l'agencement d'électrodes est commandée en fonction du profil de pression.

14. Programme informatique selon la revendication 13, dans lequel les signaux RF sont dans une plage de 0,1 MHz à 50 MHz.

15. Programme informatique selon la revendication 13 ou 14, dans lequel le procédé comprend la commande de l'alimentation des signaux RF en fonction :

de signaux de commande appliqués à la source de pression ; ou
d'une pression détectée par un capteur de pression ; ou
du fait que le profil de pression est un premier profil d'un mode de stimulation ou un second profil d'un mode d'expression ; ou
d'une qualité de contact détectée entre l'agencement d'électrodes et le sein ; ou
de signaux de capteurs acoustiques qui détectent le fonctionnement de la source de pression.

EP 4 504 283 B1

FIG. 1

FIG. 2

11

FIG. 3

FIG. 4

FIG. 5

FIG. 6

FIG. 7

FIG. 8

**EP 4 504 283 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180147331 A1
- US 20180289874 A1
- US 20200078503 A1